# EUROPEAN PATENT APPLICATION

(11) **EP 1 371 705 A1**
(43) Date of publication of application: **17.12.2003**
(21) Application number: 03253682.3
(22) Date of filing: 11.06.2003
(51) Int. Cl.: C09J 11/08, C09J 157/00

(54) **Water-based adhesives**

(30) Priority: 14.06.2002 US 389043 P; 30.09.2002 US 414590 P
(71) Applicant: ROHM AND HAAS COMPANY, Philadelphia, Pennsylvania 19106-2399 (US)
(72) Inventor: Lofton, Elizabeth P., Ambler, Pennsylvania 19002 (US); Lester, Christopher L., Telford, Pennsylvania 18969 (US); Lorah, Dennis Paul, Lansdale, Pennsylvania 19446 (US); Devonport, Wayne, Doylestown, Pennsylvania 18901 (US)
(74) Representative: Kent, Venetia Katherine

(57) **Abstract**

Improved water-based adhesives, which include polymeric nanoparticles (PNPs), are disclosed. The PNPs have polymerized units of at least one multiethylenically unsaturated monomer and of at least one ethylenically unsaturated water soluble monomer, and have a mean diameter of 1 to 50 nanometers. Preferably, the adhesive comprises at least one aqueous emulsion polymer and PNPs.

## Description

This invention relates to improved water-based or aqueous adhesives, including pressure sensitive adhesives (PSAs), dry-bond laminating adhesives, heat seal adhesives, cold seal adhesives, and barrier adhesives. In particular, this invention relates to aqueous adhesives including polymeric nanoparticles (PNPs) formed from monomers comprising multiethylenically unsaturated monomers and polymerized ionic monomers, and having mean particle diameter of from 1 to 50 nanometers (nm). The adhesives have improved properties relative to adhesives formed without the PNP.

Published PCT Patent Application WO 200075244 discloses binding agents formed by reacting one or more epoxide-functional binding agents with carboxyl functional metal-organic nanoparticles having a mean particle size of 5 to 200nm.

U.S. Patent No. 4,845,149 describes making PSAs in a resin supported polymerization using conventional dispersants. In contrast, the present invention uses PNPs as dispersants for PSAs and other adhesives.

U.S. Patent No. 6,420,023 discloses PSAs with an average particle size of about 100 nm or less, formed from a mixture of ethylenically unsaturated monomers and oligomers including one or more of N-(iso-butoxymethyl) acrylamide, N-(n-butoxymethyl) acrylamide, and N-methylol acrylamide, and emulsified in the presence of a specified emulsifier. The PSA may contain less than about 1 weight percent of 1,3-butanediol dimethacrylate monomer.

An object of the present invention is to provide aqueous adhesive compositions having at least one of the following properties improved relative to that of an adhesive absent the PNPs; in the wet composition, improved rheology, foam reduction, drying time, mechanical stability and improved wet film integrity (i.e., green strength); in the dried composition, improved specific adhesion, peel-build characteristics, water whitening resistance, and plasticizer and solvent migration resistance, gas-barrier properties, improved rheology for low temperature processing, and reduced edge ooze and flow to impart improved converting, cutting and stripping to the adhesive article. These and other objectives may be achieved by the adhesive compositions of the present invention.

In one embodiment of the invention, there is provided an aqueous adhesive comprising from 0.1 to 100 weight percent, based on a total dry weight of the adhesive, of polymeric nanoparticles (PNPs) having a mean particle diameter of 1 to 50 nm and comprising, as polymerized units, at least one multiethylenically unsaturated monomer and at least one ethylenically unsaturated water soluble monomer. In a second embodiment, the adhesive comprises from 90 to 100 wt.% PNPs, based on the total dry weight of the adhesive.

In a third embodiment of the invention, there is provided an adhesive comprising from 0.1 to 90 wt.% PNPs, based on the total dry weight of the adhesive. The adhesive may further comprise at least one emulsion polymer with a glass transition temperature (Tg) of -100 to +50°C. In one embodiment, the polymerization of monomers to form the emulsion polymer is performed in the presence of the PNPs.

In a fourth embodiment of the invention, there is provided an adhesive comprising from 10 to 60 wt.% PNP, based on the total dry weight of the adhesive. The adhesive may further comprise at least one emulsion polymer with a Tg in the range of -100 to +50°C, wherein polymerization of monomers to form the emulsion polymer is performed in the presence of the PNPs.

In a fifth embodiment of the invention, there is provided an adhesive wherein one or more of the multiethylenically unsaturated monomer and one or more of the ethylenically unsaturated water soluble monomer are the same monomer or monomers.

The aqueous compositions of the present invention are suitable for use in conventional and specialized applications calling for aqueous adhesives. In a sixth embodiment of the invention, the adhesive is used as an adhesive selected from the group consisting of: a pressure sensitive adhesive, a heat seal adhesive, a cold seal adhesive and a laminating adhesive. In a seventh embodiment of the invention, the adhesive composition has gas or moisture barrier properties.

In an eighth embodiment of the invention, there is provided an article comprising a first substrate and a layer of adhesive on at least a portion of one surface of the first substrate, the adhesive comprising from 0.1 to 100 wt.%, based on the total dry weight of the adhesive, of polymeric nanoparticles (PNPₛ) having a mean particle diameter of 1 to 50 nm and comprising, as polymerized units, at least one multiethylenically unsaturated monomer and at least one ethylenically unsaturated water soluble monomer. The article may further comprise at least one other substrate, different from the first substrate, adhered to the adhesive.

In a ninth embodiment of the invention, there is provided a release layer, having a pair of opposing surfaces, with different specific adhesion properties on the opposing surfaces, the release layer comprising at least one emulsion polymer and polymeric nanoparticles (PNPs) having a mean particle diameter of 1 to 50 nm, the PNPs comprising, as polymerized units, at least one multiethylenically unsaturated monomer and at least one ethylenically unsaturated water soluble monomer.

The aqueous adhesive composition of the present invention includes an aqueous dispersion of polymeric particles having a mean diameter in the range of from 1 to 50 nm, the particles including, as polymerized units, at least one multiethylenically unsaturated monomer and at least one ethylenically unsaturated water soluble monomer. As used herein, the term "dispersion" refers to a physical state of matter that includes at least two distinct phases wherein a first phase is distributed in a second phase, the second phase being a continuous medium. By "aqueous" herein is meant a medium that is from 50 to 100 wt.% water, based on the weight of the aqueous medium.

The polymeric particles, referred to herein as polymeric nanoparticles (PNPs), are addition polymers, which contain, as polymerized units, at least one multiethylenically unsaturated monomer and at least one ethylenically unsaturated water soluble monomer. Suitable multiethylenically unsaturated monomers useful in the present invention include di-, tri-, tetra-, or higher multifunctional ethylenically unsaturated monomers, such as, for example, divinyl benzene, trivinylbenzene, divinyltoluene, divinylpyridine, divinylnaphthalene divinylxylene, ethyleneglycol di(meth)acrylate, trimethylolpropane tri(meth)acrylate, diethyleneglycol divinyl ether, trivinylcyclohexane, allyl (meth)acrylate, diethyleneglycol di(meth)acrylate, propyleneglycol di(meth)acrylate, 2,2-dimethylpropane-1,3-di(meth)acrylate, 1,3-butylene glycol di(meth)acrylate, 1,4-butanediol di(meth)acrylate, 1,6-hexanediol di(meth)acrylate, tripropylene glycol di(meth)acrylate, triethylene glycol di(meth)acrylate, tetraethylene glycol di(meth)acrylate, polyethylene glycol di(meth)acrylates, such as polyethylene glycol 200 di(meth)acrylate and polyethylene glycol 600 di(meth)acrylate, ethoxylated bisphenol A di(meth)acrylate, poly(butanediol) di(meth)acrylate, pentaerythritol tri(meth)acrylate, trimethylolpropane triethoxy tri(meth)acrylate, glyceryl propoxy tri(meth)acrylate, pentaerythritol tetra(meth)acrylate, dipentaerythritol monohydroxypenta(meth)acrylate, divinyl silane, trivinyl silane, dimethyl divinyl silane, divinyl methyl silane, methyl trivinyl silane, diphenyl divinyl silane, divinyl phenyl silane, trivinyl phenyl silane, divinyl methyl phenyl silane, tetravinyl silane, dimethyl vinyl disiloxane, poly(methyl vinyl siloxane), poly(vinyl hydro siloxane), poly(phenyl vinyl siloxane), and mixtures thereof. The term "(meth)acrylic" includes both acrylic and methacrylic and the term "(meth)acrylate" includes both acrylate and methacrylate. Likewise, the term "(meth)acrylamide" refers to both acrylamide and methacrylamide. "Alkyl" includes straight chain, branched and cyclic alkyl groups.

Typically, the PNPs contain at least 1% by weight based on the weight of the PNPs, of at least one polymerized multiethylenically unsaturated monomer. Up to and including 99.5 wt.% polymerized multiethylenically unsaturated monomer, based on the weight of the PNPs, is effectively used in the particles of the present invention. It is preferred that the amount of polymerized multiethylenically unsaturated monomer is from 1% to 80%, more preferably from 1% to 60%, most preferably from 1% to 25%, by weight based on the weight of the PNPs.

The PNPs further contain, as polymerized units, at least one water soluble monomer. By "water soluble monomer" herein is meant a monomer having a solubility in water of at least 7 wt.%, preferably at least 9 wt.%, and more preferably at least 12 wt.%, at a temperature of 25 °C. Data for the water solubility of monomers is found, for example, in Polymer Handbook, 2^{nd} Ed., J. Brandrup, E.H. Immergut, Eds., John Wiley & Sons, New York, and Merck Index, 11^{th} Ed., Merck & Co., Inc., Rahway, NJ. Examples of water soluble monomers include ethylenically unsaturated ionic monomers and ethylenically unsaturated water soluble nonionic monomers. Typically, the amount of the polymerized water soluble monomer is at least 0.5 wt.%, based on the weight of the PNPs. Up to and including 99 wt.% polymerized water soluble monomer, based on the weight of the PNPs, can be effectively used in the particles of the present invention.

Ethylenically unsaturated ionic monomer, referred to herein as "ionic monomer" is a monomer that is capable of bearing an ionic charge in the aqueous medium in which the PNPs are dispersed. Suitable ionic monomers include, for example, acid-containing monomers, base-containing monomers, amphoteric monomers; quaternized nitrogen-containing monomers, and other monomers that can be subsequently formed into ionic monomers, such as monomers which can be neutralized by an acid-base reaction to form an ionic monomer. Suitable acid groups include carboxylic acid groups and strong acid groups, such as phosphorus containing acids and sulfur containing acids. Suitable base groups include amines. It is preferred that the amount of polymerized ionic monomer based on the weight of the PNPs is in the range from 0.5 to 99 wt.%, more preferably in the range of from 1 to 50 wt.%, even more preferably from 2 to 40 wt.%, and most preferably from 3 to 25 wt.%.

Suitable carboxylic acid-containing monomers include carboxylic acid monomers, such as (meth)acrylic acid, acryloxypropionic acid, and crotonic acid; dicarboxylic acid monomers, such as itaconic acid, maleic acid, fumaric acid, and citraconic acid; and monomers which are half esters of dicarboxylic acids, such as monomers containing one carboxylic acid functionality and one C₁₋₆ ester. Preferred are acrylic acid and methacrylic acid. Suitable strong acid monomers include sulfur acid monomers, such as 2-acrylamido-2-methyl propane sulfonic acid, styrene sulfonic acid, vinyl sulfonic acid, sulfoethyl (meth)acrylate, sulfopropyl (meth)acrylate, 2-acrylamido-2-methyl propane sulfinic acid, styrene sulfinic acid, and vinyl sulfinic acid; and phosphorus acid monomers, such as 2-phosphoethyl (meth)acrylate, vinyl phosphoric acid, and vinyl phosphinic acid. Other acid monomers include terminally unsaturated acid containing macromonomers as disclosed in U.S. Patent No. 5,710,227. Phosphorus acid monomers are desirable as they can provide improved adhesion to certain substrates (e.g., metal).

Suitable base-containing monomers include monomers having amine functionality, which includes N,N-dimethylaminoethyl (meth)acrylate, N,N-diethylaminoethyl (meth)acrylate, N-t-butylaminoethyl (meth)acrylate, N,N-dimethylaminopropyi (meth)acrylamide, p-aminostyrene, N,N-cyclohexylallylamine, allylamine, diallylamine, dimethylallylamine, N-ethyldimethylallylamine, crotyl amines, and N-ethylmethallylamine; monomers having pyridine functionality, which includes 2-vinylpyridine and 4-vinylpyridine) monomers having piperidine functionality, such as vinylpiperidines; and monomers having imidazole functionality, which includes vinyl imidazole. Other suitable base-containing monomers include oxazolidinylethyl (meth)acrylate, vinylbenzylamines, vinylphenylamines, substituted diallylamines, 2-morpholinoethyl (meth)acrylate, methacrylamidopropyl trimethyl ammonium chloride, diallyl dimethyl ammonium chloride, 2-trimethyl ammonium ethyl methacrylic chloride, and the like.

Suitable amphoteric monomers include N-vinylimidazolium sulfonate inner salts and N,N-Dimethyl-N-(3-methacrylamidopropyl)-N-(3-sulfopropyl) ammonium betaine.

Suitable functional monomers, in which the functionality is subsequently formed into an acid or base include monomers containing: an epoxide functionality, such as glycidyl (meth)acrylate and allyl glycidyl ether; an anhydride, such as maleic anhydride; an ester such as methyl acrylate; and a halide. Suitable halide-containing functional monomers include vinylaromatic halides and halo-alkyl(meth)acrylates. Suitable vinylaromatic halides include vinylbenzyl chloride and vinylbenzyl bromide. Other suitable functional monomers include allyl chloride, allyl bromide, and (meth)acrylic acid chloride. Suitable halo-alkyl(meth)acrylates include chloromethyl (meth)acrylate. Suitable functional monomers, in which the functionality is subsequently formed into a nonionic water soluble group, include vinyl acetate. Hydrolysis of the polymerized vinyl acetate provides hydroxyl groups to the PNPs.

Multiethylenically unsaturated monomers that are also water soluble monomers are alternatively used to prepare the PNPs. In such embodiments, these monomers are classified for the purposes of the present invention as both a multiethylenically unsaturated monomer and a water soluble monomer. An example of a water soluble, multiethylenically unsaturated monomer is phosphodi(ethyl methacrylate).

Ethylenically unsaturated water soluble nonionic monomers are referred to herein as "water soluble nonionic monomers". Examples of water soluble nonionic monomers include hydroxyalkyl (meth)acrylates such as hydroxyethyl (meth)acrylate and hydroxypropyl (meth)acrylate; poly(alkylene oxide) esters of (meth)acrylic acid such as poly(ethylene oxide)20 methacrylate and poly(propylene oxide)₁₅₀ acrylate; acrylamide; and methacrylamide. It is preferred that the amount of polymerized water soluble nonionic monomer based on the weight of the PNPs is in the range from 0.5 to 99 wt.%, more preferably in the range of from 20 to 90 wt.%, even more preferably from 30 to 80 wt.%, and most preferably from 40 to 70 wt.%. When the PNPs include, as polymerized units, ionic monomer and nonionic water soluble monomer, lower levels of polymerized nonionic water soluble monomer are preferred.

The PNPs optionally contain, as polymerized units, one or more third monomers that are not multiethylenically unsaturated monomers and are not water soluble monomers. Suitable third monomers include C₁-C₂₄ alkyl (meth)acrylates, such as methyl (meth)acrylate, ethyl (meth)acrylate, propyl (meth)acrylate, butyl (meth)acrylate, isobutyl (meth)acrylate, hexyl (meth)acrylate, cyclohexyl (meth)acrylate, 2-ethylhexyl (meth)acrylate, octyl (meth)acrylate, decyl (meth)acrylate, dodecyl (meth)acrylate, pentadecyl (meth)acrylate, hexadecyl (meth)acrylate, octadecyl (meth)acrylate, and nonadecyl (meth)acrylate, and mixtures thereof. Other suitable third monomers include vinyl acetate; vinyl versatate; diisobutylene; ureido containing monomers such as N-(ethyleneureidoethyl)-4-pentenamide, N-(ethylenethioureido-ethyl)-10-undecenamide, butyl ethyleneureido-ethyl fumarate, methyl ethyleneureido-ethyl fumarate, benzyl N-(ethyleneureido-ethyl) fumarate, and benzyl N-(ethyleneureido-ethyl) maleamate; vinylaromatic monomers, such as styrene, α-methylstyrene, vinyltoluene, *p*-methylstyrene, ethylvinylbenzene, vinylnaphthalene, vinylxylenes, and nonylphenoxy propenyl polyethoxylated alcohol. The vinylaromatic monomers also include their corresponding substituted counterparts, such as halogenated derivatives, i.e., containing one or more halogen groups, such as fluorine, chlorine or bromine; and nitro, cyano, (C₁-C₁₀)alkoxy, halo(C₁-C₁₀)alkyl, (C₁-C₁₀)alkoxy, carboxy, and the like. Substituted ethylene monomers useful as unsaturated monomers in the present invention include, but are not limited to: allylic monomers, vinyl acetate, vinyl versatate, vinyl formamide, vinyl chloride, vinyl fluoride, vinyl bromide, vinylidene chloride, vinylidene fluoride and vinylidene bromide.

The PNPs have a mean diameter in the range of from 1 to 50 nm, preferably in the range of from 1 to 40 nm, more preferably from 1 to 30 nm, even more preferably from 1 to 25 nm, even further preferably from 1 to 20 nm, and most preferably from 1 to 10 nm. It is further typical that the PNPs have a mean particle diameter of at least 1.5 nm, preferably at least 2 nm. One method of determining the particle sizes (mean particle diameter) of the PNPs is by using standard dynamic light scattering techniques, wherein the correlation functions are converted to hydrodynamic sizes using LaPlace inversion methods, such as CONTIN.

Typically, the PNPs including, as polymerized units, less than 10 wt.% multiethylenically unsaturated monomer, have a glass transition temperature (Tg) from -90°C to 170°C for the composition in the absence of the polymerized multiethylenically unsaturated monomer, as determined by a modulated differential scanning calorimetry (DSC) measurement. PNPs containing, as polymerized units, at least 50 wt.% multiethylenically unsaturated monomer are considered to have Tgs of at least 50°C.

The PNPs of the present invention typically have an "apparent weight average molecular weight" in the range of 5,000 to 1,000,000, preferably in the range of 10,000 to 500,000 and more preferably in the range of 15,000 to 100,000. As used herein, "apparent weight average molecular weight" reflects the size of the PNP particles using standard gel permeation chromatography methods, e.g., using THF solvent at 40 °C, 3 plgel™ Columns (Polymer Labs, Amherst, MA), 100 Angstroms (10 nm), 10³ Angstroms (100 nm), 10⁴ Angstroms (1 micron), 30 cm long, 7.8 mm ID, 1 ml per minute, 100 microliter injection volume, calibrated to narrow polystyrene standards using Polymer Labs CALIBRE ^{TM} software.

The PNPs are optionally characterized as having suitable hydrophilicities that allow the PNPs to be dispersed into an aqueous medium. One method to characterize the hydrophilicity of the PNPs is to calculate the Hansch parameter, which is actually a measure of hydrophobicity. The Hansch parameter is calculated using a group contribution method. The monomer units forming the polymer are assigned a hydrophobicity contribution and the relative hydrophobicity of the polymer is calculated based on the weight average of the monomers in the polymer. Hansch and Fujita, J. Amer. Chem. Soc., 86, 1616-1626 (1964); H. Kubinyi, Methods and Principles of Medicinal Chemistry, Volume 1, R. Mannhold et al., Eds., VCH, Weinheim (1993); C. Hansch and A. Leo, Substituent Constants for Correlation Analysis in Chemistry and Biology, Wiley, New York (1979); and C. Hansch, P. Maloney, T. Fujita, and R. Muir, Nature, 194. 178-180 (1962).

Values of the hydrophobicity contributions for several monomers are listed in Table 1.

**Table 1**

| Monomer | Hydrophobicity Contribution |
|---|---|
| ethyl acrylate | 2.11 |
| butyl acrylate | 3.19 |
| 2-ethyl hexylacrylate | 5.22 |
| styrene | 4.29 |
| methyl methacrylate | 1.89 |
| ethyl methacrylate | 2.43 |
| butyl methacrylate | 3.51 |
| isobornyl methacrylate | 5.0 |
| butadiene | 4.0 |
| acrylic acid | -2.52 |
| methacrylic acid | -2.2 |
| maleic anhydride | -3.5 |

Preferred PNPs have a Hansch parameter in the range of from -2.5 to 4, alternatively from -1 to 3.

The PNPs optionally contain other functional groups, which are provided by the polymerization of monomers containing those groups or precursor groups thereof. Functional groups are optionally attached to the PNPs by reacting the ionic group of the PNP with a suitable compound. For example, PNPs containing carboxylic acid groups are modified to contain pendant hydrophilic groups by reacting carboxylic acid groups with a suitable alcohol, such as a capped polyalkylene oxide. Alternatively, functional groups are affixed to the PNPs through non-radical reactions resulting in the formation of ionic or covalent bonds between a modifying compound containing the groups and complementary reactable groups covalently bound to the PNP as taught in U.S. Patent No. 5,270,380.

The complementary reactable groups in the PNP and modifying compound provide ionic or covalent bonding. Complementary ionic bonding includes acid-base interaction and ion pair bonding of negatively and positively charged atoms. Covalent bonding by complementary reactable groups includes, for example:
(a) acetoacetate-aldehyde; (b) acetoacetate-amine; (c) amine-aldehyde; (d) amine-anhydride; (e) amine-isocyanate; (f) amine-epoxy; (g) aldehyde-hydrazide; (i) acid-epoxy; (j) acid-carbodiimide; (k) acid-chloro methyl ester; (j) acid-chloro methyl amine; (m) acid-anhydride; (n) acid-aziridine; (o) epoxy-mercaptan; and (p) isocyanate-alcohol. The first or second reactable group in each pair is present either in the PNP or, alternatively, in the modifying compound.

A suitable method to prepare the aqueous composition containing the PNPs dispersed in an aqueous medium includes the steps of preparing a nonaqueous PNP dispersion containing the PNPs dispersed in at least one solvent; and combining the nonaqueous PNP dispersion with an aqueous medium. By "nonaqueous" herein is meant a medium that contains from zero to less than 50 wt.% water, based on the weight of the nonaqueous medium. Aqueous compositions containing PNPs that include, as polymerized units, ionic monomers, are optionally partially or completely neutralized prior to, during, or after combining with the aqueous medium.

A suitable polymerization process to prepare the nonaqueous PNP dispersion is free radical solution polymerization of at least one multiethylenically unsaturated monomer, at least one water soluble monomer, and optionally, at least one third monomer. By "solution polymerization" herein is meant free radical addition polymerization in a suitable solvent for the polymer. By "suitable solvent for the polymer" herein is meant that linear random (co)-polymers having substantially similar polymerized monomer units to the PNPs, are soluble in the solvent. Another method for selecting a suitable solvent or mixture of solvents is on the basis of using solubility parameter analysis. According to such methods, the suitability of the solvent is determined by substantially matching the solubility parameters of the PNP and of the solvent, such as the Van Krevelen parameters of delta d, delta p, delta h and delta v. See, for example, Van Krevelen et al., Properties of Polymers. Their Estimation and Correlation with Chemical Structure, Elsevier Scientific Publishing Co. (1976); Olabisi et al., Polymer-Polymer Miscibility, Academic Press, NY (1979); Coleman et al., Specific Interactions and the Miscibility of Polymer Blends, Technomic (1991); and A. F. M. Barton, CRC Handbook of Solubility Parameters and Other Cohesion Parameters, 2^{nd} Ed., CRC Press (1991). Delta d is a measure of dispersive interactions, delta p is a measure of polar interactions, delta h is a measure of hydrogen bonding interactions, and delta v is a measure of both dispersive and polar interactions. Such solubility parameters are alternatively calculated, such as by the group contribution method, or determined experimentally, as is known in the art. A preferred solvent has a delta v parameter within 5 (joule per cubic centimeter)^{½}, preferably within 1 (joule per cubic centimeter)^{½} of the polymer delta v parameter. Suitable solvents for the polymerization include organic solvents, such as hydrocarbons; alkanes; halohydrocarbons; chlorinated, fluorinated, and brominated hydrocarbons; aromatic hydrocarbons; ethers; ketones; esters; alcohols; and mixtures thereof. Particularly suitable solvents, depending on the composition of the PNP, include dodecane, mesitylene, xylenes, diphenyl ether, gamma-butyrolactone, ethyl acetate, ethyl lactate, propyleneglycol monomethyl ether acetate, caprolactone, 2-heptanone, methylisobutyl ketone, acetone, methyl ethyl ketone, diisobutylketone, propyleneglycol monomethyl ether, and alkylalcohols, such as isopropanol, decanol, and t'butanol; and supercritical carbon dioxide.

The nonaqueous PNP dispersion is prepared by first charging a solvent, or alternatively, a mixture of solvent and some portion of the monomers, to a reaction vessel. The monomer charge is typically composed of monomers, an initiator, and a chain transfer agent, if utilized. Typically, initiation temperatures are in the range of from 55°C to 125°C, although lower or higher initiator temperatures are possible using suitable low temperature or high temperature initiators known in the art. After the heel charge has reached a temperature sufficient to initiate polymerization, the monomer charge or balance of the monomer charge is added to the reaction vessel. The monomer charge time period is typically in the range of from 15 minutes to 4 hours, although both shorter and longer time periods are envisioned. During the monomer charge, the reaction temperature is typically kept constant, although it is possible to vary the reaction temperature. After completing the monomer mixture addition, additional initiator in solvent can be charged to the reaction and/or the reaction mixture may be held for a time.

Control of PNP particle size and distribution is achieved by one or more of such methods as choice of solvent, choice of initiator, total solids level, initiator level, type and amount of multi-functional monomer, type and amount of ionic monomer, type and amount of chain transfer agent, and reaction conditions.

Initiators useful in the free radical polymerization to form the PNPs include, for example, one or more of: peroxyesters, alkylhydroperoxides, dialkylperoxides, azoinitiators, persulfates, redox initiators and the like. The amount of the free radical initiator used is typically from 0.05 to 10% by weight, based on the weight of total monomer. Chain transfer reagents are optionally used to control the extent of polymerization of the PNPs useful in the present invention. Suitable chain transfer agents include, for example: alkyl mercaptans, such as dodecyl mercaptan; aromatic hydrocarbons with activated hydrogens, such as toluene; and alkyl halides, such as bromotrichloroethane.

In one method of preparing the aqueous PNP composition, at least a portion of the polymerized ionic monomer units of the PNPs are neutralized with at least one neutralizing agent to form an at least partially neutralized nonaqueous PNP dispersion. The polymerized ionic monomer units of the PNPs can be neutralized in a variety of ways. When the polymerized ionic monomer units are acidic, the neutralizing agent is typically a base. Likewise, when the polymerized ionic monomer units are basic, the neutralizing agent is typically an acid. Suitable bases include inorganic and organic bases. Suitable inorganic bases include the full range of the hydroxide, carbonate, bicarbonate, and acetate bases of alkali or alkaline metals. Suitable organic bases include ammonia, primary/secondary/tertiary amines, diamines, and triamines. Preferred basic neutralizing agents include sodium hydroxide, and ammonium hydroxide. Suitable acids include carboxylic acids, such as acetic acid; dicarboxylic acids; (di)carboxylic/hydroxyl acids; aromatic acids, such as benzoic acid; and a variety of other acids, such as boric, carbonic, citric, iodic, nitrous, nitric, periodic, phosphoric, phosphorous, sulfuric, sulfurous, and hydrochloric acid. None of the foregoing categories of bases and acids, are deemed to be limiting.

The amount of neutralizing agent required to neutralize the nonaqueous PNP dispersion is typically determined on a molar basis of neutralizing agent to polymerized ionic monomer units of the PNPs. Without being bound to a particular theory, the amount of polymerized ionic monomer units (i.e., level of charge) needed to stabilize the PNPs (i.e., maintain particle size during conversion from non-aqueous to aqueous medium) will vary as PNP composition and properties are varied. It is believed that the PNP hydrophobicity, Tg, crosslinking level, and type of counter-ion from the neutralizing agent are important variables. For providing stable aqueous PNP dispersions (i.e., wherein flocculation of the PNPs is minimized), the polymerized ionic monomer units are preferably at least 20%, more preferably at least 50%, even more preferably at least 80%, and most preferably at least 90% neutralized.

Neutralizing the PNPs is alternatively carried out in a variety of ways. In one method, the nonaqueous PNP dispersion is added to a solution containing the neutralizing agent while stirring. Preferably, the neutralizing agent is added as an aqueous solution over time while stirring the nonaqueous PNP dispersion to provide an at least partially neutralized nonaqueous PNP dispersion.

In one method of preparing the aqueous composition containing dispersed PNPs, the at least partially neutralized nonaqueous PNP dispersion is combined with an aqueous medium. The aqueous medium optionally contains the neutralizing agent(s) for neutralizing the PNPs, in which case the nonaqueous PNP dispersion is capable of being simultaneously neutralized and combined with an aqueous medium. The aqueous medium optionally contains surfactants, which are capable of altering the stability of the PNPs, or of altering other properties of the resulting aqueous PNP dispersion, such as its surface tension.

The sequence of admixing the partially neutralized nonaqueous PNP dispersion and the aqueous medium is not critical. Various methods and equipment, which are suitable for mixing are described in The Chemical Engineer's Handbook, 5^{th} Ed., Perry and Chilton, Eds., McGraw-Hill, Ch. 21 (1973). Typically, the aqueous medium is continuously stirred while adding the partially neutralized nonaqueous PNP dispersion to it in order to ensure that the solvent is intimately mixed with the aqueous medium, which minimizes flocculation of the PNPs.

Suitable weight percentages of the PNPs in the aqueous PNP composition (i.e., the range of PNP solids in the aqueous medium), based on total weight of the aqueous composition, are typically from 1 to 90 wt.%, more typically from 2 to 75 wt.%, even more typically from 4 to 65 wt.%, further more typically from 8 to 55 wt.%, and most typically from 10 to 45 wt.%.

While the preparation of the aqueous PNP composition does not require the use of surfactants, and it is typical that the nonaqueous PNP dispersions are substantially free of surfactants, surfactants are optionally included. When present, the amount of surfactants is typically less than 3 wt.%, more typically less than 2 wt.%, even more typically less than 1 wt.%, further typically less than 0.5 wt.%, and even further typically less than 0.2 wt.%, based on total weight of the PNPs.

The aqueous PNP composition is optionally treated to remove at least a portion of the solvent and optionally water, to increase the solids content of the PNPs. Suitable methods to concentrate the PNPs include distillation processes, such as forming azeotropes of water and a suitable solvent; evaporation of solvent or water; drying the aqueous composition by freeze drying or spray drying; solvent extraction techniques; and ultrafiltration techniques. Preferably at least 25 wt.%, more preferably at least 50 wt.%, even more preferably at least 75 wt.%, and most preferably 100 wt.% of the solvent is exchanged with water. Removal of the solvent is preferably carried out under conditions that minimize destabilization (i.e., flocculation) of the PNPs.

In an alternative method, the aqueous PNP composition is prepared by a method including the steps of preparing a nonaqueous PNP dispersion containing the PNPs dispersed in at least one solvent that is both a suitable solvent for the PNPs and is compatible or miscible in water; and combining the nonaqueous PNP dispersion with an aqueous medium. Examples of such suitable solvents for acrylic-containing PNPs, which are also compatible or miscible with water, include isopropanol and ether alcohols (e.g., monobutyl ether of ethylene glycol and monoethyl ether of diethylene glycol). In this method, the PNPs do not require the addition of neutralizing agents to impart particle stability when combined with water.

Alternate embodiments of the aqueous PNP compositions of the present invention have a wide range of PNP content; i.e., PNP solids in the PNP dispersion. Typically, the PNP weight fractions range from 0.1 to 99 wt.%, more typically from 1 to 90 wt.%, even more typically from 2 to 75 wt.%, further typically from 5 to 50 wt.%, and most typically 10 to 40 wt.%, based on the total weight of the aqueous PNP composition.The aqueous adhesive compositions of the present invention optionally contain other polymer particles that are not PNPs, such as emulsion polymer particles having a mean particle diameter of greater than 50 nm. The PNPs are present in the aqueous composition at a level of from 0.1 to 100 wt.% PNPs based on the total weight of the polymer particles in the aqueous composition. The functional role of the PNPs determines the level of PNPs present.

Some of the functional roles of the PNPs are, for example, as additives, as dispersants, as blend agents, and as a PNP matrix with the PNPs as principal or sole binder polymeric component. The amount of PNPs utilized ranges from a lower level for the former to a higher level of the latter.

In one embodiment, the PNPs are provided with other polymer particles, wherein the other polymer particles are prepared in the presence of the PNPs. In this embodiment, the PNPs are present in the aqueous composition at a level of from 10 to 60 wt.%, and most preferably from 20 to 40 wt.%, based on the total dry weight of polymer in the aqueous adhesive composition. When a dispersion of emulsion polymer in PNPs is formed, the resulting composition may be referred to as an resin supported emulsion polymer (ReSEP).

The PNPs can be used as a dispersion in the polymerization solvent or they can be isolated by, for example, vacuum evaporation, by precipitation into a non-solvent, and spray drying. When isolated, PNPs can be subsequently redispersed in a medium appropriate for incorporation into an adhesive composition. It is preferred for the medium to be water. Alternatively, the isolated PNPs could be redispersed directly into the water-based emulsion.

Aqueous adhesive compositions comprising PNPs of the present invention have at least one of the following properties improved relative to that of an adhesive absent the PNPs; in the wet composition, improved rheology, foam reduction, drying time, mechanical stability and improved wet film integrity (i.e., green strength); in the dried composition, improved specific adhesion, peel-build characteristics, water whitening resistance, and plasticizer and solvent migration resistance, gas-barrier properties, improved rheology for low temperature processing, and reduced edge ooze and flow to impart improved converting, cutting and stripping to the adhesive article. Depending on its properties, the adhesive composition may have application, for example, as a pressure sensitive adhesive, heat seal adhesive, cold seal adhesive, dry-bond laminating adhesive, or a gas or moisture barrier adhesive.

The aqueous adhesive compositions of the present invention comprise 0.1 to 100 wt.% PNPs based on the total dry weight of the adhesive. In preparing the adhesive composition, the PNPs are typically used as the aqueous PNP composition described above with a solid content from 0.1 to 99 wt.% of the PNP based on the total weight of the aqueous PNP adhesive. The adhesive may be formed from one, or more than one aqueous PNP composition.

In one embodiment, the aqueous adhesive composition comprises PNPs as an additive or blend agent; that is, the PNPs are admixed or blended with an emulsion polymer to form the adhesive composition. In this embodiment, the adhesive composition comprises from 0.1 to 90 wt.% PNPs based on the total dry weight of the adhesive. Preferably, the adhesive composition comprises 1 to 90 wt.%., more preferably 5 to 50 wt.%, and still more preferably 10 to 40 wt.% of the PNP compositions. At lower levels, the PNP may be considered an additive, while at high levels it may considered a blend agent.

In another embodiment, the aqueous adhesive composition of the present invention comprises from 90 to 100 wt.% PNPs. The adhesive may comprise the PNP composition as the sole polymer component of the adhesive. It is noted that the PNP composition itself may comprise other components as described elsewhere above. Where the adhesive comprises less than 100 wt.% of PNPs, the remainder of the adhesive composition may comprise emulsion polymer or other components admixed or blended with the PNP. When the adhesive is formed from 100% of one or more PNP compositions, the dried adhesive will contain 100 wt.% PNPs based on the total dry weight of the adhesive.

In another embodiment, the aqueous adhesive composition of the present invention comprises from 10 to 60 wt.% PNPs, preferably 20 to 40 wt.% PNPs, and the emulsion polymer of the adhesive composition is polymerized from suitable monomers in the presence of the PNP composition. This forms a ReSEP adhesive. In this embodiment, the PNPs are provided with other polymer particles, wherein the other polymer particles are prepared in the presence of the PNPs. In this embodiment, the PNPs are present in the aqueous composition at a level of from 1 to 99 wt.% solids, preferably from 5 to 90 wt.%, more preferably from 10 to 60 wt.%, and most preferably from 20 to 40 wt.%, based on the total dry weight of polymer in the aqueous composition.

In this embodiment, PNPs of the present invention can be used as stabilizers (i.e., dispersants) in emulsion polymerizations according to the methods known for using "high acid" polymeric stabilizers (often referred to as "resin supported emulsion polymerization", see for example U.S. Patent No. 6,020,061), or for using steric stabilizers. Among suitable emulsion polymer compositions, any emulsion polymer, copolymer, multi-stage copolymer, interpolymer, core-shell polymer, and the like can be stabilized using the PNPs of the the present invention. While any ethylenically unsaturated monomer may be used, it is preferred that the emulsion polymers which are stabilized are prepared from at least one of (meth)acrylic ester.

In carrying out emulsion polymerizations containing the PNP stabilizers of the present invention, all of the typical emulsion polymerization components, conditions, and processes can be used, e.g., any known emulsion polymerization emulsifier (soap) may be present (or even absent), initiators, temperatures, chain transfer agents, reactor types and solids content, and the like. Latex prepared in this manor are believed to have PNPs on the latex particle surface as well as in the aqueous phase. This results in improvements in wet and dry properties of the adhesive. Also, such PNP stabilized latexes can display enhanced colloidal stability relative to analogous blends of PNPs and conventional latex.

As is readily apparent, for each of these aqueous adhesive compositions, the total amount of PNP solids present in the resulting adhesive, and consequently the amount of PNP in the dried adhesive, is a function of both the weight percent of aqueous PNP composition used in making the aqueous adhesive composition and the weight percent of polymeric nanoparticles in the aqueous PNP composition.

The emulsion polymers used in the adhesive of the present invention are prepared by emulsion polymerization techniques well known in the art. The emulsion polymer may be formed from any monomer or mixture of monomers which yields a water-insoluble latex, film-forming polymer. The emulsion polymers used in the aqueous adhesives of this invention typically have a glass transition temperature as measured via differential scanning calorimetry in the range from -100 to +50°C. For PSAs, the Tg is preferably in the range from -70 to -10°C, more preferably, -70 to -35°C. For dry bond laminating adhesives, the Tg is preferably in the range from -50 to +50°C, more preferably, -30 to +25°C, still more preferably, -10 to +10°C. For heat seal adhesives, the Tg is preferably in the range from -20 to +50°C, more preferably, +10 to +30°C; additionally, the modulus for heat seal adhesives is preferably approximately 3 x 10⁶ dyne/cm at temperatures in the range from 25 to 100°C. For cold seal adhesives, the Tg is preferably in the range from -100 to +10°C, preferably, -80 to 0°C.

The Mw of the emulsion polymer may be adjusted through the addition of a chain transfer agent, such as n-dodecyl mercaptan, during emulsion polymerization to give a suitable balance of adhesive and cohesive strength.

A wide variety of monomers or mixture of monomers may be used to make the emulsion polymer used in the adhesives of this invention. For example, acrylic ester monomers, including methyl acrylate, ethyl acrylate (EA), propyl acrylate, isopropyl acrylate, butyl acrylate (BA), isobutyl acrylate, secondary butyl acrylate, t-butyl acrylate, pentyl acrylate, neopentyl acrylate, hexyl acrylate, heptyl acrylate, octyl acrylate, isooctyl acrylate, 2-ethylhexyl acrylate (EHA), decyl acrylate, isodecyl acrylate, lauryl acrylate, bornyl acrylate, isobornyl acrylate, myristyl acrylate, pentadecyl acrylate, stearyl acrylate and the like; methacrylic acid ester monomers, including methyl methacrylate, ethyl methacrylate, propyl methacrylate, isopropyl methacrylate, butyl methacrylate, isobutyl methacrylate, hexyl methacrylate, octyl methacrylate, isooctyl methacrylate, decyl methacrylate, isodecyl methacrylate, lauryl methacrylate, bornyl methacrylate, isobornyl methacrylate, myristyl methacrylate, pentadecyl methacrylate, stearyl methacrylate, phosphoethyl methacrylate and the like; acrylic acid (AA), methacrylic acid (MAA), itaconic acid, maleic acid, fumaric acid, styrene, substituted styrenes, butadiene, acrylonitrile, ethylene, vinyl acetate, vinyl chloride, sodium styrene sulfonates, sodium vinyl sulfonate, acrylamide, methacrylamide and the like may be used. Other emulsion polymers may include, but are not limited to, dispersions of polyurethanes, polyesters, natural rubber, synthetic rubbers, polyvinyl ether, polyvinyl chloride and copolymers thereof, urea formaldehyde, phenol formaldehyde, rescorcinol, and block copolymers (such as Kraton™ resins).

The adhesive composition can contain conventional adhesive adjuvants such as, for example, tackifiers, emulsifiers and wetting agents, crosslinkers, monomers, oligomers, polymers, solvents or plasticizers, buffers, neutralizers, thickeners or rheology modifiers, biocides, antifoaming or defoaming agents.

In one embodiment of the invention, the adhesive composition may comprise 90 to 100 wt.% PNP based on the total dry weight of the adhesive composition. If less than 100 wt.% PNP is utilized, the remaining weight fraction may comprise emulsion polymer, dispersant, surfactant, filler or other additives for use in the adhesive composition. In another embodiment of the invention, the adhesive composition may comprise suitable emulsion polymer blended with 0.1 to 90 wt.% PNP based on the total dry weight of the adhesive composition. At the lower end of this range, the PNP may be characterized as an additive, while at the higher end of this range, it may be characterized as a blend agent. In still another embodiment of the invention, the adhesive composition may comprise PNPs agglomerated around or supported on suitable emulsion polymer particles as a resin-supported emulsion polymer (ReSEP). Typically, the ReSEP composition contains 10 to 60 wt.% PNP based on the total dry weight of the adhesive composition. The adhesives of each such composition may be used as PSAs, laminating adhesives, heat seal and cold seal adhesives.

The solids content of the aqueous adhesive compositions of the present invention can be from about 10% to about 70% by weight. The viscosity is typically from 0.05 to 5 Pa.s (50 cps to 5000 cps), as measured using a Brookfield viscometer; the viscosities appropriate for different end uses and application methods vary considerably. The adhesive can applied by conventional adhesive application methods such as, for example, curtain coating, roller coating, reverse roller coating, meyer rod, slot die and gravure coating. The adhesive compositions can be applied to a substrate such as, for example, plastic including sheets and films of substituted ethylene monomers, polypropylene, polyethylene, polyamide, polyamide imide, polyester, or composite blends, metallized films, woven and nonwoven textiles, paper, with or without a prior substrate treatment such as a chemical primer, treated release liners, or corona or flame treated surfaces. The chemical primer can be a pre-coat of the adhesive composition with or without PNPs, or may be an alternate primer composition. The adhesive coating on the substrate is typically dried, or allowed to dry, at temperatures from 40°C to 200°C.

In one embodiment of the present invention, an aqueous adhesive containing a polymer dispersed in an aqueous medium, such as an emulsion polymer, having a particle size greater than 50 nanometers is admixed with PNPs. The aqueous adhesive so formed exhibits at least one of the improved properties, in wet or dry state, noted above, relative to that of such an aqueous adhesive composition absent the PNPs.

In one embodiment of the present invention, an aqueous adhesive containing a polymer dispersed in an aqueous medium such as an emulsion polymer having a particle size greater than 50 nm is prepared in the presence of PNPs. The polymers dispersed in an aqueous medium can be prepared by various polymerization methods, such as emulsion polymerization, mini-emulsion, micro-emulsion, suspension polymerization, non-aqueous dispersion polymerization, or solution polymerization. The aqueous adhesive so formed exhibits at least one of the improved properties, in wet or dry state, noted above, relative to that of such an aqueous adhesive composition absent the PNPs.

In one embodiment of the present invention an aqueous adhesive containing a polymer dispersed in an aqueous medium such as an emulsion polymer is admixed or blended with PNPs, the PNPs being provided as a dispersion in a solvent which is a plasticizer, or a water soluble solvent or solvent mixture compatible with the emulsion polymer. The aqueous adhesive so formed exhibits at least one of the improved properties, in wet or dry state, noted above, relative to that of such an aqueous adhesive composition absent the PNPs.

In one embodiment of the present invention, an aqueous adhesive including PNPs bearing a functional species, such as for example, an adhesion promoter or a release agent, or additional crosslinkable moiety, is provided. For example, the functional species can be provided by a corresponding functional monomer introduced in the preparation of the PNP, by post reaction of a PNP, or by physical attachment such as adsorption, hydrogen bonding, etc. of an appropriate species to a PNP. The PNPs provide a more efficient use of the functional species relative to the adhesive absent the PNPs. The PNPs can also provide a more efficient use of the functional species relative to the adhesive wherein the functional species are attached to particles larger than 50 nm.

In one embodiment, the adhesives with PNPs have useful specific adhesion, meaning improved adhesion to particular difficult to bond substrates. While not being bound to a particular theory, the PNPs in such adhesives may migrate to the air interface or coated substrate and subsequently provide specific adhesion to either the facestock (or a substrate layer) of the adhesive article or to the surface to which the adhesive article is adhered. This property may be used advantageously to provide a suitable adhesive layer in an adhesive article, or to provide a release layer, for example in an adhesive tape article. Selection of PNPs with suitable polar functional groups can be used to promote adhesion to various difficult to bind substrates, such as high energy substrates, including metallized films and paper. Hydroxyl (for example, HEMA type) and acid containing monomers and PEM are such polar monomers. Likewise, selection of PNPs with suitable non-polar functional groups can be used to promote adhesion to substrates such as low energy substrates like high and low density polyethylene, untreated oriented polypropylene (OPP) and printed olefins. Silicone and fluoromonomers, as well as other hydrophobic monomers are examples of such non-polar monomers.

In one embodiment of the present invention, an adhesive containing a polymer dissolved in aqueous solution is admixed with PNPs including at least one copolymerized surface-active monomers, such as fluoromonomers, silicon-containing monomers, or poly(alkylene-oxide)-containing monomers having poly(alkylene-oxide) segment molecular weights in the range of 100 to 5,000 g/mol (such as monomers including poly(ethylene oxide) segments), or mixtures thereof. In this embodiment, the weight percent of surface-active monomers used for preparing the PNPs is in the range from 1 to 90%, preferably from 2 to 75%, more preferably from 5 to 50%, and even more preferably from 10 to 40%. PNPs containing fluorine, silicon, and poly(alkylene-oxide) groups can also be prepared by reacting PNPs containing reactive groups with fluorine, silicon, and poly(alkylene oxide) chemical moieties that chemically interact with the reactive groups of the PNPs. The dry adhesive formed from such adhesive compositions exhibits improved specific adhesion or release.

Aqueous adhesive compositions with desirable gas or moisture barrier properties can be prepared using PNPs which comprise suitable monomers such as vinyl chloride, vinylidene chloride, vinyl alcohol, acrylonitrile and other monomers known to provide barrier functionality when incorporated in suitable polymers.

The following examples are presented to illustrate further various aspects of the present invention.

### EXAMPLE 1. Preparation of Butyl Acrylate PNPs.

A 5 liter reactor was fitted with a thermocouple, a temperature controller, a purge gas inlet, a water-cooled reflux condenser with purge gas outlet, a stirrer, and an addition funnel. To the addition funnel was charged 571.5 g of a monomer mixture consisting of 337.5 g butyl acrylate (100% purity), 67.5 g acrylic acid (100% purity), 45 g trimethylol propane triacrylate, 9 g of a 75% solution of t-amyl peroxypivalate in mineral spirits (Luperox 554-M-75), and 112.5 g isopropyl alcohol ("IPA"). The reactor, containing 2334 g IPA was then flushed with nitrogen for 30 minutes before applying heat to bring the contents of the reactor to 75°C. When the contents of the reactor reached 75°C, the monomer mixture in the addition funnel was uniformly charged to the reactor over 90 minutes. Thirty minutes after the end of the monomer mixture addition, the first of two chaser aliquots, spaced thirty minutes apart and consisting of 9.0 g of a 75% solution of t-amyl peroxypivalate in mineral spirits (Luperox 554-M-75) and 23 g IPA, was added. At the end of the second chaser aliquot, the contents of the reactor were held 2½ hours at 80°C to complete the reaction. The resulting polymer was isolated removal of solvent *in vacuo.* This material was alkaline water. The PNPs thus formed had a particle size distribution of from 2.8 to 3.6 nm as determined by GPC.

### EXAMPLE 2. Preparation of Butyl Acrylate PNPs.

A 5 liter reactor was fitted with a thermocouple, a temperature controller, a purge gas inlet, a water-cooled reflux condenser with purge gas outlet, a stirrer, and an addition funnel. To the addition funnel was charged 612.75 g of a monomer mixture consisting of 356.25 g butyl acrylate (100% purity), 71.75g acrylic acid (100% purity), 47.5 g trimethylol propane triacrylate, 19 g of a 75% solution of t-amyl peroxypivalate in mineral spirits (Luperox 554-M-75), and 118.75 g methylisobutyl ketone ("MIBK"). The reactor, containing 2454 g MIBK was then flushed with nitrogen for 30 minutes before applying heat to bring the contents of the reactor to 75°C. When the contents of the reactor reached 75°C, the monomer mixture in the addition funnel was uniformly charged to the reactor over 90 minutes. Thirty minutes after the end of the monomer mixture addition, the first of two chaser aliquots, spaced thirty minutes apart and consisting of 9.5 g of a 75% solution of t-amyl peroxypivalate in mineral spirits (Luperox 554-M-75) and 23 g MIBK, was added. At the end of the second chaser aliquot, the contents of the reactor were held 2½ hours at 80°C to complete the reaction. The resulting polymer was isolated removal of solvent *in vacuo.* This material was alkaline water. The PNPs thus formed had a particle size distribution of from 6 to 8 nm as determined by GPC.

The PNPs of Examples 1 and 2 were mixed with commercial PSA emulsion Rhoplex™ N-619 (Rohm and Haas Company, Philadelphia) at 5, 10 and 20 parts per hundred emulsion (PHE). The pH was kept above 7.5 with ammonia. The emulsion mixture was equilibrated overnight, before being coated directly or transferred from release liner to 2 mil polyester film. The adhesive was dried for 3 minutes at 100°C in a conventional forced air oven, forming a dry film of 0.9 mils (coat weight = cwt.). The adhesive article was closed to release liner. The loop tack, 90° peel and shear (1" x 1" x 1 kg) on stainless steel (ss) panels was measured.

| | | | Post addition of Neut. JR-5488 (30% solids) | | | | |
|---|---|---|---|---|---|---|---|
| | | | | | | | |

| Mylar, direct coat | | | Peel Value | | Shear | | Tack |
|---|---|---|---|---|---|---|---|
| cwt. = 0.9 mils | | | (N / in²) | | (hours) | | (N/ in) |
| | | | (ss) | | (ss) | | (ss) |
| | | | | | | | |
| N-619 | Control | | 10.5, A | | 51.7, C | | 17.0, A |
| | | | | | | | |
| N-619/ 5 phe, neut.JR-5488 (30%) | | | 12.5, A | | 29.8, C | | 16.5, A |
| | | | | | | | |
| N-619 / 10 phe, neut.JR-5488 (30%) | | | 12.1, A | | 24.9, C | | 14.4, A |
| | | | | | | | |
| N-619 / 10 phe, neut.JR-5488 (30%) | | | 12.5, A | | 24.6, C | | 14.5, A |
| Dried @ 4.5 min. | | | | | | | |
| | | | | | | | |
| N-619 /20 phe, neut.JR-5488 (30%) | | | 11.8, A | | 14.0, C | | 12.0, A |
| | | | | | | | |

The mode of failure is indicated above as either adhesive failure ("A") or cohesive failure ("C").

Additionally the mechanical stability of the wet adhesive compositions was tested in comparison to the wet Rhoplex N-619 emulsion alone via finger rub. The PNPs significantly improved the mechanical stability of the wet adhesive, and a pass rating was given to adhesive blends containing 10 and 20 PHE above. The wet adhesives were also evaluated for foaming tendency, and the compositions with PNPs had substantially lower foam.

### EXAMPLE 3. Preparation of a PNP Suitable for Resin Supported Emulsion Polymerization.

PNPs of methyl methacrylate/ butyl acrylate / acrylic acid / trimethylol propane triacrylate (35/35/20/10 wt.%) were prepared via solution polymerization as follows: A 5 liter reactor was fitted with a thermocouple, a temperature controller, a purge gas inlet, a water-cooled reflux condenser with purge gas outlet, a stirrer, and a monomer feed line. To a separate vessel was charged 450.0 g of a monomer mixture (A) consisting of 157.5 g methyl methacrylate (MMA), 157.5 g butyl acrylate (BA), 90.0 g acrylic acid (AA), and 45.00 g trimethylol propane triacrylate (TMPTA). To an additional vessel was charged an initiator mix (B) consisting of 18.00 g of a 75% solution of t-amyl peroxypivalate in mineral spirits (Triganox 125-C75), and 112.50 g isopropyl alcohol. A charge of 2325.00 g isopropyl alcohol was added to the reactor. After sweeping the reactor with nitrogen for approximately 30 minutes, heat was applied to bring the reactor charge to 79°C. When the contents of the reactor reached 79°C, a dual feed of both the monomer mixture (A) and the initiator mix (B) were added to the reactor. The two mixtures were fed uniformly using feed pumps over 120 minutes. At the end of the monomer and initiator feeds, the batch was held at 79°C for 30 minutes before adding the first of three additional initiator charges consisting of 9.00 g of a 75% solution of t-amyl peroxypivalate in mineral spirits (Triganox 125-C75), and 22.50 g isopropyl alcohol. A second initiator charge addition was made 30 minutes after the first initiator charge addition. Similarly, the final initiator charge addition was made 30 minutes after the second initiator charge addition. The batch was then held at the polymerization temperature of 79°C for and additional 2½ hours to achieve full conversion of monomer. At the end of the final hold, the batch was neutralized with a mixture of 42.5 gm of an aqueous 28% solution of aqueous ammonia and 450.00 g water. The neutralized polymer solution was transferred to a roto-evaporator and stripped of solvent at ∼35°C at reduced pressure. After removing all solvent the batch was further diluted with water to ∼25% polymer (PNP) in water. Particle size was measured at ∼5.0 nm. The resulting aqueous PNP dispersion can be used as a stabilizer for emulsion polymerizations.

### EXAMPLE 4. Resin Supported Emulsion Polymerization Utilizing a PNP.

342 g (25.6% active in water) of ammonia neutralized acrylic acid based PNPs, pH 8-9 adjusted with aqueous ammonia (35MMA/35BA/20AA/10TMPTA, particle size less than 10 nm; prepared according to Example 3) were added to a 2-liter, 4 neck round bottom flask equipped with a side arm, condenser, stirrer, and thermocouple. The flask contents was heated to 85°C under a nitrogen sweep. The monomer, 350.0 g of butyl acrylate, was added over a 1.5 hour period, while simultaneously adding a separate solution of 2.63 g ammonium persulphate in 100.0 g DI water and 0.22 g of 28% ammonium hydroxide, over a 2 hour time period. After the monomer mix feed was complete the persulphate cofeed continued for another 30 minutes. At this point, the contents of the flask was held at 85°C for an additional 60 minutes. Afterwards, the contents of the flask was cooled to 25°C and filtered through a 100/325 mesh set of stacked screens, yielding a negligible quantity of coagulated polymer. The resulting filtered emulsion polymerization product had a solids content of 55.04%, pH 7.52, particle size of 128 nm and a viscosity of 1090 cps. This latex contained 20% PNP and 80% poly butyl acrylate.

### EXAMPLE 5. Resin Supported Emulsion Polymerization Utilizing a PNP.

Following the procedure of Example 4, another resin supported emulsion polymer was prepared utilizing 25% of the same PNP and 75% of poly butyl acrylate. The resulting filtered emulsion polymerization product had a solids content of 52.87%, pH 7.49, particle size of 148 nm and a viscosity of 950 cps.

### COMPARATIVE EXAMPLE A. Resin Supported Emulsion Polymerization Utilizing a Conventional Dispersant.

Following the procedure of Example 4, another resin supported emulsion polymer was prepared utilizing 20% of a conventional dispersant and 80% of butyl acrylate. The conventional dispersant was Joncryl™ 678 (S.C. Johnson & Sons, Inc., Racine, WI). The solid material can be dissolved in aqueous ammonia and utilized identically to the PNP in Example 4 for a resin supported emulsion polymerization, except that 9.45 g of a nonionic surfactant, Macol 1 (BASF Corp.) is required to produce a stable product. It is mixed with the butyl acrylate monomer. The resulting filtered emulsion polymerization product had a solids content of 49.02%, pH 7.84 , particle size of 158 nm and a viscosity of 160 cps.

### COMPARATIVE EXAMPLE B. Resin Supported Emulsion Polymerization Utilizing a Conventional Dispersant.

Following the procedure of Comparative Example A, another resin supported emulsion polymer was prepared utilizing 25% of the same conventional dispersant and 75% of butyl acrylate. The resulting filtered emulsion polymerization product had a solids content of 48.19%, pH 7.97, particle size of 142 nm and a viscosity of 260 cps.

### EXAMPLES 6-7. PSA with PNPs.

The resin supported polymers of Examples 4 and 5 made with 20% and 25% PNPs, respectively, were both direct and transfer coated to 2 mil polyester film and dried 3 minutes at 100°C to a film thickness of 0.8 mils. Additionally, the resin supported polymers made with the conventional dispersant Joncryl™ 678 in Comparative Examples A and B were coated and dried in the same manner. The poly butyl acrylate (pBA) adhesives made with 20% or 25% PNPs had measurable loop tack, 90° peel and shear (1" x 1" x 1kg) on stainless steel, whereas those made with 20% and 25% Joncryl 678 with pBA had no measurable tack, peel or shear.

| | | **Adhesives** | **Made With** | **PNP** |
|---|---|---|---|---|
| | | **Peel (N/in**^{**2**}**)** | **Shear (hours)** | **Tack (N/in.)** |
| **DAK-1561** | Direct Coat | 3.6, A | 6.6, C | 5.3, A |
| **20% pBA** | Transfer Coat | 3.1, A | 100, C | 6, A |
| | | | | |
| **DAK-1563** | Direct Coat | 0.3, A | 2.3, C | 2.2, A |
| **25% pBA** | Transfer Coat | 2.4, A | 1.5, C | 2.9, A |

The mode of failure is indicated above as either adhesive failure ("A") or cohesive failure ("C").

### EXAMPLE 8. Dry Bond Laminating with Lower Nip Temperature.

A PNP of composition 70 BA/ 20 AA/ 10 TMPTA, with 40-60% solids, is prepared according to Example 1. A formulation is formed containing 5% based on solids of this PNP and 95% based on solids of a polymer emulsion of composition 98.2 BA/ 1.8 MAA, an emulsion that alone is not used in laminating applications. The formulation is prepared by providing the polymer emulsion in a vessel, adjusting the pH to 9 by dropwise addition of 14% by volume of NH₄OH while stirring with a benchtop mixer, and then adding the PNP dropwise with continued stirring. The preparation is mixed for approximately 10 minutes, capped, and then allowed to equilibrate for one hour.

For laminating, the formulation is drawn down onto a 1.5 mil oriented polypropylene (OPP) film using a #1 wire wound rod to yield a wet film thickness of approximately 0.2 mil. The coated film is placed in a 104°C oven for 3 minutes to dry the film to a thickness of approximately 0.1 mil. The dried film is removed from the oven and the adhesive is contacted with another 1.5 mil OPP film, applying a pressure with a rubber roller. The OPP-OPP laminate is then run through a set of rollers at a pressure of approximately 20 psi at a temperature of 38°C. The resulting laminate has a bond strength of 250 g/in at 25°C and 65°C from t-peel adhesion tests performed at 10 in./min. In addition to providing good adhesion, the PNP increases shear strength strength. The nip temperature of 38°C is considerably lower than the 85°C used in conventional systems.

### EXAMPLE 9. Cold Seal Adhesive with PNPs.

A blend of polymer emulsion (98.2 BA/ 1.8 MAA) and PNP (75 MMA/ 20 AA / 5 TMPTA) is prepared according to the process of Example 10. The adhesive has little tack based on a finger touch test. Two 1.5 mil OPP films are coated with a 0.1 mil coating of the adhesive. The two adhesive surfaces are then contacted to one another at room temperature to form a laminate. The bond strength is in the range from 300 to 500 g/in, as measured by a 90° peel test.

### EXAMPLE 10:

PNPs of methyl methacrylate/ methacrylic acid /trimethylol propane triacrylate (70/20/10 wt. %) were prepared via solution polymerization as follows: A 5 liter reactor was fitted with a thermocouple, a temperature controller, a purge gas inlet, a water-cooled reflux condenser with purge gas outlet, a stirrer, and a monomer feed line. To a separate vessel was charged 450.00 g of a monomer mixture (A) consisting of 315.00 g methyl methacrylate (MMA), 90.00 gm methacrylic acid (MAA), and 45.00 gm trimethylol propane triacrylate (TMPTA). To an additional vessel was charged an initiator mix (B) consisting of 18.00 g of a 75% solution of t-amyl peroxypivalate in mineral spirits (Triganox 125-C75), and 112.50 g isopropyl alcohol. A charge of 2325.00 gm isopropyl alcohol was added to the reactor. After sweeping the reactor with nitrogen for approximately 30 minutes, heat was applied to bring the reactor charge to 79°C. When the contents of the reactor reached 79°C, a dual feed of both the monomer mixture (A) and the initiator mix (B) to the reactor. The two mixtures were feed uniformly using feed pumps over 120 minutes. At the end of the monomer and initiator feeds, the batch was held at 79°C for 30 minutes before adding the first of three initiator chasers consisting of 9.00 g of a 75% solution of t-amyl peroxypivalate in mineral spirits (Triganox 125-C75), and 22.50 g isopropyl alcohol. A second initiator chaser addition was made 30 minutes after the first initiator chaser addition. Similarly, the final initiator chaser addition was made 30 minutes after the second initiator chaser addition. The batch was then held at the polymerization temperature of 79°C for and additional 2½ hours to achieve full conversion of monomer. At the end of the final hold, the batch was neutralized with a mixture of 42.5 gm of an aqueous 50% solution of NH₄OH and 450.00 gm water. The neutralized polymer solution was transferred to a roto-evaporator and stripped of solvent at ∼35°C under full house vacuum. After removing all solvent the batch was further dilution with water to ∼40% polymer (PNP) in water. Particle size was measured at ∼5.0 nm. The resulting aqueous PNP dispersion can be used as a stabilizer for emulsion polymerizations.

### EXAMPLE 11.

295.3 g of deionized water was added to a 2-liter, 4 neck round bottom flask equipped with a side arm, condenser, stirrer, and thermocouple. 160.6 g (51.6% active in water) of ammonia neutralized acrylic acid based PNPs, pH 8-9 (70MMA/20MAA/10TMPTA, particle size less than 10 nm; prepared according to Example 10) were then added to the round bottom flask and used as a stabilizer. The flask contents was heated to 85°C under a nitrogen sweep and then 6.8 g of a monomer mix consisting of 145.9 g of styrene, 185.7 g of 2-ethyl hexyl acrylate and 0.35 g of butyl mercaptopropionate was added. Immediately after adding the 6.8 g of monomer mix to the flask an ammonium persulfate solution (0.33 g of ammonium persulfate dissolved in 3 g of di-ionized water) was added to the flask and the contents of the flask is held at 85°C for 15 minutes. After the 15 minute hold an additional ammonium persulfate solution (1.0 gram of ammonium persulfate dissolved in 17.8 g of water) was added to the flask and the remaining monomer mix is fed to the flask over 150 minutes. Sixty minutes into the monomer mix feed an ammonium persulfate cofeed solution (1.2 g of ammonium persulfate dissolved in 29.9 g of water) was added to the flask over 120 minutes. 140 minutes into the monomer mix feed the reaction temperature is increased to 87°C. Upon completion of the monomer mix feed the contents of the flask was held at 87°C for an additional 60 minutes. Afterwards, the contents of the flask was cooled to 25°C and filtered through a 100/325 mesh set of stacked screens, yielding a negligible quantity of coagulated polymer. The resulting filtered emulsion polymerization product had a solids content of 48.1%, pH 8.3, particle size of 700 nm and a viscosity of 1,340 cps.

### EXAMPLE 12 to 24. PNPs used in Emulsion Polymerizations.

### EXAMPLE 12:

PNPs of methyl methacrylate/ methacrylic acid /trimethylol propane triacrylate (70/20/10 wt.%) were prepared via solution polymerization as follows: A 5 liter reactor was fitted with a thermocouple, a temperature controller, a purge gas inlet, a water-cooled reflux condenser with purge gas outlet, a stirrer, and a monomer feed line. To a separate vessel was charged 450.00 g of a monomer mixture (A) consisting of 315.00 g methyl methacrylate (MMA), 90.00 g methacrylic acid (MAA), and 45.00 g trimethylol propane triacrylate (TMPTA). To an additional vessel was charged an initiator mix (B) consisting of 18.00 g of a 75% solution of t-amyl peroxypivalate in mineral spirits (Triganox 125-C75), and 112.50 g isopropyl alcohol. A charge of 2325.00 g isopropyl alcohol was added to the reactor. After sweeping the reactor with nitrogen for approximately 30 minutes, heat was applied to bring the reactor charge to 79°C. When the contents of the reactor reached 79°C, a dual feed of both the monomer mixture (A) and the initiator mix (B) to the reactor. The two mixtures were feed uniformly using feed pumps over 120 minutes. At the end of the monomer and initiator feeds, the batch was held at 79°C for 30 minutes before adding the first of three initiator chasers consisting of 9.00 g of a 75% solution of t-amyl peroxypivalate in mineral spirits (Triganox 125-C75), and 22.50 g isopropyl alcohol. A second initiator chaser addition was made 30 minutes after the first initiator chaser addition. Similarly, the final initiator chaser addition was made 30 minutes after the second initiator chaser addition. The batch was then held at the polymerization temperature of 79°C for and additional 2½ hours to achieve full conversion of monomer. At the end of the final hold, the batch was neutralized with a mixture of 42.5 g of an aqueous 50% solution of NH₄OH and 450.00 g water. The neutralized polymer solution was transferred to a roto-evaporator and stripped of solvent at ∼35°C under full house vacuum. After removing all solvent the batch was further dilution with water to ∼40% polymer (PNP) in water. Particle size was measured at ∼5.0 nm. The resulting aqueous PNP dispersion can be used as a stabilizer for emulsion polymerizations.

### EXAMPLE 13.

295.3 g of deionized water was added to a 2-liter, 4 neck round bottom flask equipped with a side arm, condenser, stirrer, and thermocouple. 160.6 g (51.6% active in water) of ammonia neutralized acrylic acid based PNPs, pH 8-9 (70MMA/20MAA/10TMPTA, particle size less than 10 nm; prepared according to Example 12) were then added to the round bottom flask and used as a stabilizer. The flask contents was heated to 85°C under a nitrogen sweep and then 6.8 g of a monomer mix consisting of 145.9 g of styrene, 185.7 g of 2-ethyl hexyl acrylate and 0.35 g of butyl mercaptopropionate was added. Immediately after adding the 6.8 g of monomer mix to the flask an ammonium persulfate solution (0.33 g of ammonium persulfate dissolved in 3 g of di-ionized water) was added to the flask and the contents of the flask is held at 85°C for 15 minutes. After the 15 minute hold an additional ammonium persulfate solution (1.0 g of ammonium persulfate dissolved in 17.8 g of water) was added to the flask and the remaining monomer mix is fed to the flask over 150 minutes. Sixty minutes into the monomer mix feed an ammonium persulfate cofeed solution (1.2 g of ammonium persulfate dissolved in 29.9 g of water) was added to the flask over 120 minutes. 140 minutes into the monomer mix feed the reaction temperature is increased to 87°C. Upon completion of the monomer mix feed the contents of the flask was held at 87°C for an additional 60 minutes. Afterwards, the contents of the flask was cooled to 25°C and filtered through a 100/325 mesh set of stacked screens, yielding a negligible quantity of coagulated polymer. The resulting filtered emulsion polymerization product had a solids content of 48.1%, pH 8.3, particle size of 700 nm and a viscosity of 1,340 cps.

### EXAMPLES 14-24.

The PNPs listed in the following table are prepared according to the method of Example 12 and are used in emulsion polymerizations according to Example 13. Utilizing PNPs of different compositions results in different particle sized latices and variations on the improved properties they exhibit. The resulting emulsion polymers are used for formulating adhesives which have improved plasticizer migration resistance, improved solvent migrated resistance, improved gas-barrier properties, or improved specific adhesion over comparable emulsion polymers prepared without these PNPs.

### Table of PNPs Useful as Emulsion Stabilizers

| **Example** | **Composition** | **Particle Size (nm)** |
|---|---|---|
| 14 | 70 MMA / 20 MAA /10 TMPTA | 10 |
| 15 | 80 MMA / 10 AA/ 10 TMPTA | 10 |
| 16 | 75 MMA / 20 AA / 5 ALMA | 8 |
| 17 | 35 MMA / 35 BA / 20 AA / 10 TMPTA | 8 |
| 18 | 30 MMA / 30 BA / 30 AA / 10 TMPTA | 10 |
| 19 | 60 BA / 30 AA / 10 TMPTA | 10 |
| 20 | 20 MMA / 40 2-EHA / 30 AA / 10 TMPTA | 10 |
| 21 | 30 Sty / 30 MMA / 20 AA / 10 TMPTA /10 AAEM | 10 |
| 22 | 70 MMA / 20 PEM /10 TMPTA | 15 |
| 23 | 20 BA / 60 AA / 20 TMPTA | 15 |
| 24 | 80 AA / 20 TMPTA | 20 |

## Claims

1. An aqueous adhesive comprising from 0.1 to 100 weight percent, based on a total dry weight of the adhesive, of polymeric nanoparticles (PNPs) having a mean particle diameter of 1 to 50 nanometers and comprising, as polymerized units, at least one multiethylenically unsaturated monomer and at least one ethylenically unsaturated water soluble monomer.

2. The adhesive of claim 1 comprising from 90 to 100 weight percent PNPs, based on the total dry weight of the adhesive.

3. The adhesive of claim 1 comprising from 0.1 to 90 weight percent PNPs, based on the total dry weight of the adhesive.

4. The adhesive of claim 3 further comprising at least one emulsion polymer with a glass transition temperature (Tg) of-100 to +50°C.

5. The adhesive of claim 4 whereing polymerization of monomers to form the emulsion polymer is performed in the presence of the PNPs.

6. The adhesive of claim 1 comprising from 10 to 60 weight percent PNP, based on the total weight of the adhesive.

7. The adhesive of claim 6 further comprising at least one emulsion polymer with a Tg in the range of-100 to +50°C, wherein polymerization of monomers to form the emulsion polymer is performed in the presence of the PNPs.

8. The adhesive of claim 1 wherein one or more of the multiethylenically unsaturated monomer and one or more of the ethylenically unsaturated water soluble monomer are the same monomer or monomers.

9. The adhesive of claim 1, wherein the adhesive is used as an adhesive selected from the group consisting of: a pressure sensitive adhesive, a heat seal adhesive, a cold seal adhesive and a laminating adhesive.

10. An adhesive with gas or moisture barrier properties comprising the adhesive of claim 1.

11. An article comprising a first substrate and a layer of adhesive on at least a portion of one surface of the first substrate, the adhesive comprising from 0.1 to 100 weight percent, based on the total dry weight of the adhesive, of polymeric nanoparticles (PNPs) having a mean particle diameter of 1 to 50 nanometers and comprising, as polymerized units, at least one multiethylenically unsaturated monomer and at least one ethylenically unsaturated water soluble monomer.

12. The article according to claim 11, further comprising at least one other substrate, different from the first substrate, adhered to the adhesive.

13. A release layer, having a pair of opposing surfaces, with different specific adhesion properties on the opposing surfaces, the release layer comprising at least one emulsion polymer and polymeric nanoparticles (PNPs) having a mean particle diameter of 1 to 50 nanometers, the PNPs comprising, as polymerized units, at least one multiethylenically unsaturated monomer and at least one ethylenically unsaturated water soluble monomer.
